(19) Europäisches Patentamt
European Patent Office
Office européen des brevets

(11) **EP 1 701 161 B1**

(12) **EUROPÄISCHE PATENTSCHRIFT**

(45) Veröffentlichungstag und Bekanntmachung des Hinweises auf die Patenterteilung:
**05.10.2011  Patentblatt 2011/40**

(51) Int Cl.:
*G01N 27/414* (2006.01)     *G01N 21/53* (2006.01)

(21) Anmeldenummer: **06110733.0**

(22) Anmeldetag: **06.03.2006**

(54) **Brandmelder mit Streustrahlungssensor und Gassensor**

Fire detector having an optical sensor and a gas sensor

Détecteur d'incendie avec un capteur optique et un capteur de gaz

(84) Benannte Vertragsstaaten:
**CH DE FR GB LI**

(30) Priorität: **08.03.2005  DE 102005010454**

(43) Veröffentlichungstag der Anmeldung:
**13.09.2006  Patentblatt 2006/37**

(73) Patentinhaber: **ROBERT BOSCH GMBH**
**70442 Stuttgart (DE)**

(72) Erfinder: **Schumann, Bernd**
**71277, Rutesheim (DE)**

(56) Entgegenhaltungen:
**EP-A1- 0 926 646     EP-A1- 1 376 505**
**EP-A2- 1 104 884     DE-A1- 4 445 359**
**DE-A1- 19 850 564    DE-C1- 19 512 126**
**US-A- 4 103 997**

## Beschreibung

Stand der Technik

Die Erfindung bezieht sich auf einen Gassensor gemäss dem Oberbegriff des Anspruchs 1.

Um eine möglichst frühzeitige und zuverlässige Detektion von Bränden zu ermöglichen, werden Gassensoren als Brandmelder benötigt, die Brandgase schon im kleinsten Konzentrationen detektieren können. So ist beispielsweise aus der DE 199 56 303 A1 ein Brandmelder bekannt, dessen Messprinzip auf einem Feldeffekttransistor (FET) beruht, dessen Gateelektrode mindestens eine gassensitive Schicht aufweist, sodass sich die Kanalleitfähigkeit des Feldeffekttransistors in Abhängigkeit von der Konzentration eines oder mehrerer zu detektierender Brandgase verändert.

Der in der DE 199 56 303 A1 beschriebene Brandmelder kann neben einer Messfunktion auf der Basis eines FET zusätzlich einen Streulichtdetektor enthalten. Dabei ist eine Lichtquelle vorgesehen, die üblicherweise Licht mit einer Wellenlänge im Infrarot- oder Nahen Infrarotbereich in eine Messkammer aussendet, die mit der Umgebungsatmosphäre in Kontakt steht In der Messkammer ist weiterhin eine Detektoreinheit vorgesehen, die eine Bestimmung von ggf. in der Gasphase enthaltenen Partikeln ermöglicht. Ein derartiger Aufbau mit einer Lichtquelle, einem Detektor und einer Messfunktion in Form eines FET als jeweils separate Bauelemente ist jedoch aufwendig.

Ein Brandmelder mit einem Meldereinsatz ist aus der Druckschrift EP 1 376 505 A1 bekannt. Dieser weist eine Sensoranordnung und eine Auswerteelektronik mit einem die Sensoranordnung umgebenden Gehäuse mit Öffnungen für den Zutritt von Luft und gegebenenfalls Rauch zur Sensoranordnung auf. Der Melder ist modular aufgebaut und zur Aufnahme von Detektionsmodulen mit Sensoren für verschiedene Brandkenngrössen ausgebildet, wobei alle Detektionsmodule mit einem einzigen Gehäuse kompatibel sind.

Ein Sensor zum Nachweis von brennbaren Gasen in einem Prüfgas ist in der Druckschrift DE 44 45 359 A1 beschrieben. Dieser Sensor umfasst eine auf einem isolierenden, keramischen Substrat aufgebrachte sensitiven Schicht auf der Basis eines halbleitenden Metalloxids, dessen elektrischer Widerstand eine Aussage über die Konzentration von brennbaren Gasen in einem Prüfgas liefert. Es ist vorgesehen, dass die sensitive Schicht einen Verbund von zusammengesinterten Körnern des halbleitenden Metalloxids aufweist und die Oberfläche des Verbundes mit Gold und/oder einer Goldlegierung beschichtet ist.

Die Druckschrift EP 1104 884 A2 beschreibt einen Brandmelder mit mindestens einem Gassensor, wobei zur Gasdetektion mindestens ein Feldeffekttransistor mit einem Sourcebereich, einem Kanalbereich, einer Gatelektrode und einem Drainbereich vorgesehen ist. Die Gateelektrode des Feldeffekttransistors zur Gasdetektion weist eine oder mehrere gassensitive Schichten auf, so dass die Kanalleitfähigkeit des Feldeffekttransistors in Abhängigkeit von einem oder mehreren zu detektierenden Gasen steuerbar ist.

Der vorliegenden Erfindung liegt die Aufgabe zugrunde, einen Gassensor bereitzustellen, der eine zuverlässige und frühzeitige Detektion von Bränden ermöglicht und dennoch einen einfachen Aufbau aufweist.

Vorteile der Erfindung

Der erfindungsgemäße Gassensor hat den Vorteil, dass die der Erfindung zugrundeliegende Aufgabe in vorteilhafter Weise gelöst wird. Dies beruht im wesentlichen darauf, dass mindestens zwei der zur Detektion von Bränden verwendeten Baukomponenten des Gassensors zu einer Baueinheit zusammengefasst werden. Dies ermöglicht eine Branddetektion auf mindestens zwei voneinander unabhängigen Wegen, ohne dass ein aufwendiger Aufbau des Brandmelders resultiert.

Mit den in den Unteransprüchen aufgeführten Maßnahmen sind vorteilhafte Weiterbildungen des erfindungsgemäßen Gassensors möglich.

So ist von Vorteil, wenn ein Streulichtdetektor oder eine lichtquelle des Gassensors auf einem gemeinsamen Substrat mit einem Feldeffekttransistor, einer Halbleiterdiode oder einem ohmschen Widerstand vorgesehen sind. Diese bilden auf diesem Wege eine gemeinsame und somit platzsparende Baueinheit.

Weiterhin ist von Vorteil, wenn zumindest eine Gate-Elektrode des Feldeffekttransistors eine auf Brandgase sensitive Schicht aufweist. Diese kann beispielsweise auf der Basis saurer oder basischer Oxide ausgeführt sein, sodass sie auf entsprechende basische bzw. saure Brandgase anspricht, oder auf der Basis von Zinndioxid, wobei eine Empfindlichkeit auf organische Verbrennungsprodukte resultiert. Die Existenz einer sensitiven Schicht in Kontakt mit der Gate-Elektrode des FET führt zu einem empfindlichen Detektor für Brandgase.

In einer besonders vorteilhaften Ausführungsform der vorliegenden Erfindung weist der Gassensor zwei Lichtquellen auf, die Licht unterschiedlicher Wellenlänge aussenden, wobei die Wellenlänge des von der ersten Lichtquelle ausgesandten Lichtes vorzugsweise das 1,1 bis 3fache der Wellenlänge des von zweiten Lichtquelle ausgesandten Lichtes beträgt. Auf diese Weise lassen sich erfolgreich insbesondere Partikel bzw. Aerosole mit einem Durchmesser im Bereich von 10 bis 500 nm sehr genau detektieren, da sich aus dem Verhältnis der Streuintensitäten zueinander brandtypische Partikel bzw. Aerosole von Staubteilchen der Luft unterscheiden lassen.

Zeichnung

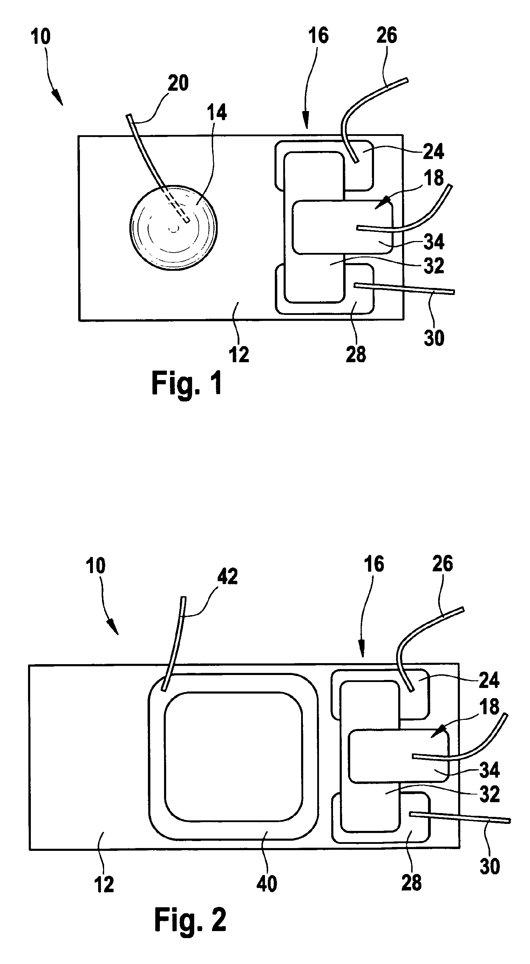

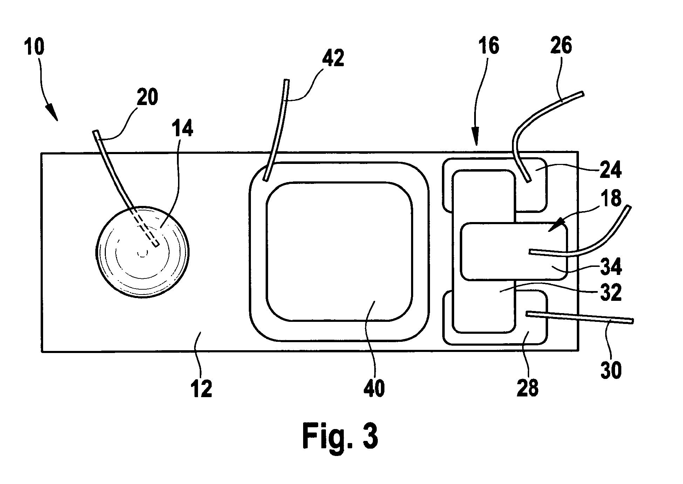

Figur 1 zeigt eine schematische Darstellung eines ersten Ausführungsbeispiels eines sensitiven Elementes eines Gassensors, Figur 2 zeigt die schematische Darstellung eines sensitiven Elementes eines Gassensors gemäß einem zweiten Ausführungsbeispiel und Figur 3 die schematische Darstellung eines sensitiven Elementes eines Gassensors gemäß einem dritten Ausführungsbeispiel.

Ausführungsbeispiele

In Figur 1 ist ein sensitives Element 10 eines Gassensors dargestellt. Das sensitive Element 10 umfasst ein Substrat 12, das beispielsweise aus Silicium, Siliciumcarbid oder Galliumnitrid, dass ggf. auf Saphir aufgebracht ist, gefertigt ist. Auf dem Substrat 12 ist eine Lichtquelle 14 und ein Feldeffekttransistor (FET) 16 angeordnet. Zusätzlich ist optional in das Substrat 12 ein nicht dargestelltes Heizelement integriert, das der zeitweiligen oder regelmäßigen Erhitzung des Substrates 12 auf eine Temperatur dient, bei der auf der Oberfläche des FET 16 adsorbierte Substanzen desorbieren und somit eine Regenerierung des sensitiven Elementes 10 bewerkstelligt werden kann. Das Heizelement kann beispielsweise als mäanderförmig ausgeführte Widerstandsleiterbahn ausgestaltet werden. Alternativ zur Aufbringung eines FET oder zusätzlich dazu kann auf dem Substrat 12 eine Halbleiterdiode oder ein ohmscher Widerstand vorgesehen sein.

Als Lichtquelle 14 wird beispielsweise eine light emitting diode (LED) gewählt, die Licht mit einer Wellenlänge von ≤ 550 nm, vorzugsweise im Bereich von 250 bis 550 nm, insbesondere im Bereich von 350 bis 550 nm aussendet. Dazu wird die LED vorzugsweise auf der Basis eines dotierten Halbleitermaterials ausgeführt, das einen Bandabstand von 2 bis ca. 5 e V aufweist. Dies sind beispielsweise Materialien wie α-oder β-Galliumnitrid, 6H- oder β-Siliciumcarbid, Zinkoxid, α- oder β-Aluminiumnitrid, α- oder β-Indiumnitrid, Zinksulfid, Zinkselenid, Aluminiumphosphid, Galliumphosphid und Aluminiumarsenid.

Die Verwendung einer dergestalt ausgeführten LED als Lichtquelle 14 hat den Vorteil, dass Licht kurzer Wellenlänge deutlich stärker an Partikeln in der Gasphase gestreut wird als Strahlung des NIR- oder IR-Bereichs. Ein weiterer Vorteil der Verwendung einer LED als Lichtquelle 14, die Licht kurzer Wellenlänge zur Verfügung stellt, ist darin zu sehen, dass eine Beheizung des Substrates 12, auf dem sich sowohl die Lichtquelle 14 als auch der FET 16 bzw. eine Halbleiterdiode oder ein ohmscher Widerstand befinden, nur bei Verwendung einer LED mit großem Bandabstand möglich ist. Andere LED, die Strahlung im NIR- bzw. IR-Bereich aussenden, können aufgrund deren erhöhter thermisch bedingter Leitfähigkeit nicht aufbeheizten Substraten eingesetzt werden. Die Lichtquelle 14 ist durch eine elektrische Kontaktierung 20 elektrisch kontaktiert.

Der FET 16 umfasst einen Sourcebereich 24, der mit einer elektrischen Kontaktierung 26 versehen ist, sowie einen Drainbereich 28, der mit einer weiteren elektrischen Kontaktierung 30 verbunden ist, wobei der Sourcebereich 24 und der Drainbereich 28 durch einen halbleitenden, ggf. dotierten Kanalbereich 32 miteinander verbunden sind. In Kontakt mit dem Kanalbereich 32 steht eine Gateelektrode 34.

Der FET 16 weist beispielsweise auf dessen Gateelektrode 34 eine gassensitive Beschichtung 18, die als Basis ein Edelmetall oder eine Edelmetalllegierung enthält, wobei Edelmetall- bzw. Edelmetalllegierungspartikel in Form von Nanopartikeln eingesetzt werden, auf, wobei die Gateelektrode 34 teil- oder vollflächig mit der gassensitiven Beschichtung 18 versehen sein kann.

Bei Verwendung dieser Substanzen kommt es insbesondere bei der Absorption von organischen Gasbestandteilen wie polykondensierten Aromaten und Kohlenwasserstoffen sowie von Kohlenmonoxid zu einer Veränderung der elektrischen Ladungsträgerverteilung in der sensitiven Beschichtung 18.

Alternativ oder zusätzlich zur Anordnung eines FET 16 kann auf dem Subtrat 12 auch eine Halbleiterdiode oder ein ohmscher Widerstand vorgesehen sein. In diesem Fall wird zumindest ein Teil der einer Umgebungsatmosphäre ausgesetzten Großfläche mit der sensitiven Beschichtung 18 versehen, wobei die sensitive Beschichtung 18 in diesem Fall aus vergleichbaren Materialien ausgeführt ist wie sie bereits vorstehend als Beschichtung für den FET 16 beschrieben wurden.

Der Gassensor umfasst weiterhin einen Detektor 40 zur Detektion der von der Lichtquelle 14 ausgesandten Strahlung. Dieser umfasst beispielsweise als Licht detektierendes Material eines der dotierten Halbleitermaterialien, wie sie vorstehend als Basismaterial der Lichtquelle 14 beschrieben wurden.

Der Detektor 40 ist so positioniert, dass von diesem lediglich eine Streustrahlung des von der Lichtquelle 14 ausgesandten Lichtes erfasst wird; er ist also insbesondere außerhalb des von der Lichtquelle 14 erzeugten Strahlungskegels angeordnet. Dabei ist der Detektor 40 beispielsweise als Photodiode, photosensitiver Widerstand oder als Phototransistor ausgeführt. Weiterhin enthält der Brandmelder einen Parabolspiegel, mit dem Streulicht gebündelt dem Detektor zugeführt wird.

Dieser Aufbau des Gassensors ermöglicht zum einen über die Detektion von Streustrahlung, die sich insbesondere bei Existenz von Ruß- bzw. Rauchpartikeln oder von Aerosolen verstärkt, bei Einsatz als Brandmelder die Erkennung eines Brandes. Darüber hinaus kann durch die Bestimmung eines den FET 16 bzw. eine Halbleiterdiode oder einen Halbleiterwiderstand durchfließenden elektrischen Stromes, dessen Höhe von u.a. bei Bränden auftretenden Gasen abhängig ist, ein Brand erkannt werden. Diese doppelte Branderkennung ermöglicht eine bedeutend größere Systemsicherheit, da sowohl stark rußende, schwach giftige Brände erkannt werden als auch schwach rußende, mit giftigen Gasen verbundene Brände. Dennoch ist der Gesamtaufbau des Gassensors nicht aufwendig, da mindestens zwei der Bauelemente 14, 16, 40 auf dem Substrat 12 des sensitiven Elementes 10 eine Baueinheit bilden.

Ein weiteres Ausführungsbeispiel eines sensitiven Elementes 10 ist in Figur 2 dargestellt, wobei gleiche Bezugszeichen gleiche Bauteilkomponenten bezeichnen wie in Figur 1.

Dabei ist auf dem Substrat 12 des sensitiven Elementes 10 anstelle der Lichtquelle 14 ein Detektor 40 für die von der Lichtquelle 14 ausgesandte Strahlung angeordnet. Der Detektor 40 ist durch eine elektrische Kontaktierung 42 kontaktiert. Die Lichtquelle 14 ist dabei separat positioniert; dies jedoch so, dass von dem Detektor 40 lediglich der gestreute Anteil des von der Lichtquelle 14 ausgesandten Lichtes detektiert wird.

Das sensitive Element 10 wird bevorzugt so ausgeführt, dass die Gateelektrode 34 und der lichtsensitive Bereich des Detektors 40 auf der Basis desselben Halbleitermaterials ausgeführt sind, da dies die Herstellung des sensitiven Elementes 10 wesentlich vereinfacht. Dabei wird als Material des Detektors 40 bspw. Galliumnitrid, Indium-Galliumnitrid, Aluminium-Galliumnitrid oder Siliciumcarbid verwendet.

Ein weiteres Ausführungsbeispiel eines sensitiven Elementes 10 ist in Figur 3 dargestellt, wobei weiterhin gleiche Bezugszeichen gleiche Bauteilkomponenten bezeichnen wie in den Figuren 1 und 2.

Wie Figur 3 zu entnehmen ist, weist das Substrat 12 des sensitiven Elementes 10 gemäß drittem Ausführungsbeispiel neben dem FET 16 sowohl eine Lichtquelle 14 als auch einen Detektor 40 auf Der besondere Vorteil dieser Anordnung besteht darin, dass alle Baukomponenten des Gassensors auf einem gemeinsamen Substrat 12 des sensitiven Elementes 10 angeordnet sind und der Gassensor selbst kostengünstig und raumsparend ausgeführt werden kann.

Eine alternative Ausführungsform der in den Figuren 1 bis 3 dargestellten sensitiven Elemente besteht darin, zwei lichtquellen 14 vorzusehen, die Strahlung verschiedener Wellenlänge erzeugen. Dabei ist insbesondere von Vorteil, wenn die weitere Lichtquelle eine Strahlung mit einer Wellenlänge erzeugt, die vorzugsweise dem 1,1 bis 3fachen, insbesondere dem 1,1 - 1,5fachen derjenigen Wellenlänge entspricht, die die der von der ersten Lichtquelle 14 erzeugte Strahlung aufweist.

Dabei werden je nach Steuerung der Lichtquellen zeitgleich oder zeitversetzt zwei Streulichtsignale mittels des Detektors 40 detektiert, die unterschiedliche Lichtintensitäten aufweisen. Theoretisch ist die gemessene Lichtintensität von der Wellenlänge der gestreuten Strahlung abhängig nach der Formel:

$$I = 1 / \lambda^4.$$

Demnach sind zwischen beiden Signalen je nach gewählten Wellenlängen der beiden Lichtquellen Intensitätsdifferenzen mit einem Faktor von 1,5 bis 5 zu erwarten. Auf diese Weise kann der Streuanteil des detektierten Lichtes sehr genau erfasst werden; insbesondere derjenige Anteil, der auf Teilchen oder Aerosole mit einer Teilchengröße von 10 - 500 nm zurückzuführen ist, wie sie bei Bränden typischerweise auftreten.

Der beschriebene Gassensor eignet sich neben einer Verwendung als Brandmelder insbesondere zur Früherkennung von Schwel- oder Kabelbränden auch als Sensor zur Bestimmung von Gaskomponenten in Abgasen von Verbrennungsmotoren, Kraftwerken etc. oder als Luftgütesensor. Weiterhin können schädliche Abgasbestandteile in den Abgasen von Müllverbrennungsanlagen detektiert werden.

**Patentansprüche**

1. Gassensor, insbesondere Brandmelder, mit einer Lichtquelle zur Erzeugung von Licht mit einer Wellenlänge < 550 nm und einem Detektor zur Bestimmung einer Streustrahlung, wobei ein Feldeffekttransistor (16), eine Halbleiterdiode oder ein Halbleiterwiderstand mit der Lichtquelle (14) und/oder dem Detektor (40) eine Baueinheit bildet, wobei der Brandmelder einen Parabolspiegel enthält, mit dem dem Detektor Streulicht gebündelt zugeführt wird, wobei zumindest eine Gate-Elektrode (34) des Feldeffekttransistors (16) oder ein Oberflächenbereich der Halbleiterdiode oder des ohmschen Widerstandes eine auf bei Bränden auftretende Gase sensitive Schicht (18) aufweist, **dadurch gekennzeichnet, dass** ein Edelmetall bzw. eine Edelmetalllegierung als Basis für die Schicht (18) verwendet wird, wobei Edelmetall- bzw. Edelmetalllegierungspartikel in Form von Nanopartikeln eingesetzt werden.

2. Gassensor nach Anspruch 1, **dadurch gekennzeichnet, dass** der Feldeffekttransistor (16), die Halbleiterdiode oder der Halbleiterwiderstand auf einem Substrat (12) angeordnet ist, auf dem sich auch die Lichtquelle (14) und/oder der Detektor (40) befindet.

3. Gassensor nach Anspruch 1, **dadurch gekennzeichnet, dass** die sensitive Schicht (18) Oxide des Tantals, Niobiums oder der Erdalkalimetalle enthält.

4. Gassensor nach Anspruch 1, **dadurch gekennzeichnet, dass** die sensitive Schicht (18) Zinndioxid enthält.

5. Gassensor nach einem der vorhergehenden Ansprüche, **dadurch gekennzeichnet, dass** zwei Lichtquellen (14) vorgesehen sind, die Licht unterschiedlicher Wellenlänge aussenden.

6. Gassensor nach Anspruch 5, **dadurch gekennzeichnet, dass** die Wellenlänge des von der ersten Lichtquelle (14) ausgesandten Lichtes das 1,1 bis 3fache der Wellenlänge des von zweiten Lichtquelle ausgesandten Lichtes beträgt.

7. Gassensor nach einem der vorhergehenden Ansprüche, **dadurch gekennzeichnet, dass** die Baueinheit ein Heizelement aufweist.

## Claims

1. Gas sensor, in particular fire alarm, comprising a light source for generating light having a wavelength of < 550 nm and a detector for determining a scattered radiation, wherein a field effect transistor (16), a semiconductor diode or a semiconductor resistor forms a structural unit with the light source (14) and/or the detector (40), wherein the fire alarm contains a parabolic mirror, by means of which scattered light is fed in a concentrated fashion to the detector, wherein at least a gate electrode (34) of the field effect transistor (16) or a surface region of the semiconductor diode or of the semiconductor resistor has a layer (18) sensitive to gases that occur in the event of fires, **characterized in that** a noble metal or a noble metal alloy is used as a basis for the layer (18), wherein noble metal or noble metal alloy particles in the form of nanoparticles are used.

2. Gas sensor according to Claim 1, **characterized in that** the field effect transistor (16), the semiconductor diode or the semiconductor resistor is arranged on a substrate (12), on which the light source (14) and/or the detector (40) are/is also situated.

3. Gas sensor according to Claim 1, **characterized in that** the sensitive layer (18) contains oxides of tantalum, niobium or of alkaline earth metals.

4. Gas sensor according to Claim 1, **characterized in that** the sensitive layer (18) contains tin dioxide.

5. Gas sensor according to any of the preceding claims, **characterized in that** two light sources (14) are provided, which emit light having different wavelengths.

6. Gas sensor according to Claim 5, **characterized in that** the wavelength of the light emitted by the first light source (14) is 1.1 to 3 times the wavelength of the light emitted by the second light source.

7. Gas sensor according to any of the preceding claims, **characterized in that** the structural unit has a heating element.

## Revendications

1. Détecteur de gaz, notamment avertisseur d'incendie, comprenant une source de lumière pour générer de la lumière avec une longueur d'onde < 550 nm et un détecteur pour déterminer un rayonnement diffusé, un transistor à effet de champ (16), une diode à semi-conducteur ou une résistance à semi-conducteur formant un sous-ensemble avec la source de lumière (14) et/ou avec le détecteur (40), l'avertisseur d'incendie contenant un miroir parabolique avec lequel la lumière diffusée est acheminée en faisceau au détecteur, au moins une électrode de gâchette (34) du transistor à effet de champ (16) ou une zone de surface de la diode à semi-conducteur ou de la résistance à semi-conducteur présentant une couche (18) sensible aux gaz produits en cas d'incendie, **caractérisé en ce qu'**un métal noble ou un alliage de métal noble est utilisé comme base pour la couche (18), des particules de métal noble ou d'alliage de métal noble étant utilisées sous la forme de nanoparticules.

2. Détecteur de gaz selon la revendication 1, **caractérisé en ce que** le transistor à effet de champ (16), la diode à semi-conducteur ou la résistance à semi-conducteur est disposé(e) sur un substrat (12) sur lequel se trouvent également la source de lumière (14) et/ou le détecteur (40).

3. Détecteur de gaz selon la revendication 1, **caractérisé en ce que** la couche sensible (18) contient de l'oxyde de tantale, de niobium ou de métaux alcalinoterreux.

4. Détecteur de gaz selon la revendication 1, **caractérisé en ce que** la couche sensible (18) contient de l'oxyde d'étain.

5. Détecteur de gaz selon l'une des revendications précédentes, **caractérisé en ce que** deux sources de lumière (14) sont prévues, lesquelles émettent de la lumière à des longueurs d'onde différentes.

6. Détecteur de gaz selon la revendication 5, **caractérisé en ce que** la longueur d'onde de la lumière émise par la première source de lumière (14) est égale à 1,1 à 3 fois la longueur d'onde de la lumière émise par la deuxième source de lumière.

7. Détecteur de gaz selon l'une des revendications précédentes, **caractérisé en ce que** le sous-ensemble présente un élément chauffant.

Fig. 1

Fig. 2

**Fig. 3**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19956303 A1
- EP 1376505 A1
- DE 4445359 A1
- EP 1104884 A2